# EUROPEAN PATENT APPLICATION

(11) **EP 1 283 054 A1**
(43) Date of publication of application: **12.02.2003**
(21) Application number: 01912425.4
(22) Date of filing: 15.03.2001
(51) Int. Cl.: A61K 45/00, A61K 31/198, A61P 3/10, A61P 25/00, A61P 9/00, A61P 13/12

(54) **DRUGS FOR COMPLICATIONS OF DIABETES AND NEUROPATHY AND UTILIZATION THEREOF**

(30) Priority: 17.03.2000 JP 2000076542
(71) Applicant: Ajinomoto Co., Inc., Tokyo 104-8315 (JP)
(72) Inventor: KITAHARA, Yoshiro, Pharmaceutical Res. Lab., Kawasaki-shi, Kanagawa 210-8681 (JP); MIURA, Kyoko, Pharmaceutical Res. Lab., Kawasaki-shi, Kanagawa 210-8681 (JP)
(74) Representative: Nicholls, Kathryn Margaret
(86) International application number: JP0102094
(87) International publication number: WO01068136

(57) **Abstract**

There are provided a medicament excellent in prevention, improvement, treatment, etc. of a diabetic complication, and a medicament excellent in prevention, improvement, treatment, etc. of a neuropathy, each using a postprandial blood sugar-lowering agent as an effective ingredient therefor. Particularly, D-phenylalanine derivatives such as nateglinide are useful as an oral medicine.

According to the present invention, these excellent medicaments, a method for use thereof (a method for administration thereof to a living subject in the above treatment, etc.), and a use of the postprandial blood sugar-lowering agent for production of the said medicaments, and the like are provided. At least one agent selected from an antihypertensive agent, a vasodilating agent and an anti-hyperlipidemic agent may be used at the same time with the postprandial blood sugar-lowering agent, by mixing further such at least one agent selected from an antihypertensive agent, a vasodilating agent and an anti-hyperlipidemic agent in (with) such above medicament or by combining them for concomitant use, whereby the effect for prevention, improvement, treatment, etc. of the diabetic complication described above can be enhanced much more.

## Description

### Technical Field

The present invention relates to novel medicaments (medical drugs) suitable for a diabetic complication and/or a neuropathy, and specifically to a medicament suitable for prevention, improvement and/or treatment of a diabetic complication comprising a postprandial blood sugar-lowering agent (postprandial hypoglycemic agent), and a medicament suitable for prevention, improvement and/or treatment of a neuropathy comprising a postprandial blood sugar-lowering agent. Further, it also relates to uses of these medicaments (such as uses of the medicaments for prevention, improvement and/or treatment of the diabetic complication or the neuropathy described above and the like), a use of the postprandial blood sugar-lowering agent described above for production of the medicaments described above, and the like.

### Background Art

Diabetes mellitus (DM) is a disease in which persistency of chronic hyperglycemia is a major sign. The number of diabetic patients is increasing all over the world, but the risk of diabetic coma (narcosis) and infections has greatly been reduced by means of insulin, antibiotics, and so on. However, even now, the diabetes mellitus is so frequently accompanied by so-called three major complications including neuropathy, retinopathy and nephropathy, which are considered as a microangiopathy; the patients, accordingly, are considerably restricted in their daily life and social activity, and have been compelled to an inconvenient life. Moreover, it is also known to be a risk factor to promote arteriosclerosis (sclerosis of the arteries) that is said to be a cause of macroangiopathy, and it becomes an important problem or target in the current therapy of diabetes mellitus that even if the diabetes mellitus occurs, these vascular complications could not be allowed to occur the onset or progress.

### Problem to be solved by Invention

At present, there is not found an excellent pharmaceutical preparation (drug medicine; medicament) suitable for prevention, improvement, or treatment of these complications.

It has been reported in the DCCT study or the UKPDS study that strict control of the blood sugar with an insulin injection or sulfonylurea agents (chlorpropamide, glibenclamide, glipizide and the like) reduces a risk of onset or progress of the complication, but occurrence of the complication has not yet been inhibited completely with these drugs. Moreover, an oral preparation currently provided for clinical use as a drug for treating a chronic complication is an aldose reductase (reducing enzyme) inhibitor alone, and it is said that its efficacy is limited; therefore, another good drug medicine (medicament), particularly as an oral preparation, is expected to be developed.

On the other hand, the neuropathy is a disorder which is caused by dysfunction of the central and peripheral nerves, including perceptive and sensorial disorder, dyskinesia, and other many neural symptoms, of which some (as complication) are caused by diabetes mellitus, and some not in many cases; there is no good pharmaceutical preparation (medicament) found suitable for these disorders, i.e., necessary for prevention, improvement, or treatment of the neuropathy.

In the above circumstances, it has been desired to develop a pharmaceutical preparation effective to the prevention, improvement, and/or treatment of the diabetic complication or the neuropathy. The problem to be solved by the present invention, that is the purpose of the present invention is to develop and provide such a pharmaceutical preparation (medicament).

### Disclosure of Invention

The present inventors diligently have spent much time for the study towards solving the above problem, and as a result, have found that a postprandial blood sugar-lowering agent (hypoglycemic agent) improves decrease of a motor nerve conduction velocity of a Goto-Kakizaki rat (hereinafter referred to as "GK rat") known as a model animal of diabetes mellitus, and accordingly, it can be used as a pharmaceutical preparation (medicament) for prevention, improvement, and/or treatment of a diabetic complication, and further it can also be used as a pharmaceutical preparation for prevention, improvement, and/or treatment of a neuropathy not caused by diabetes mellitus.

Further, the present inventors have found that the postprandial blood sugar-lowering agent restrains (suppresses) a decrease of a Na⁺/K⁺-ATPase activity in nerve which is an index (indicator) for the nerve function, that the postprandial blood sugar-lowering agent restrains an increase of vWF concentration in the blood which is an indicator of endothelial dysfunction and that the postprandial blood sugar-lowering agent lowers lipid in the blood which is a risk factor for arteriosclerosis (arteries) and the macroangiopathy.

The present invention has been completed on the basis of these various findings.

That is to say, the present invention lies in a medicament or a drug medicine as one embodiment thereof, which can be exemplified typically or representatively by the followings:
(1) A medicament for prevention, improvement and/or treatment of a diabetic complication, comprising a postprandial blood sugar-lowering agent;
(2) A medicament for prevention, improvement and/or treatment of a neuropathy, comprising a postprandial blood sugar-lowering agent; and
(3) A drug medicine suitable or usable for the medicament for prevention, improvement and/or treatment of a diabetic complication,
comprising a postprandial blood sugar-lowering agent and at least one agent selected from an antihypertensive agent, a vasodilating agent and an anti-hyperlipidemic agent; or
being or having a combination of a postprandial blood sugar-lowering agent, with at least one agent selected from an antihypertensive agent, a vasodilating agent and an anti-hyperlipidemic agent.

In the above medicament for prevention, improvement and/or treatment of diabetic complication, the effectiveness of the medicament can be enhanced greater by using the postprandial blood sugar-lowering agent;
in the mixture with at least one agent selected from an antihypertensive agent, a vasodilating agent (an agent for improving a peripheral blood flow) and an anti-hyperlipidemic agent (an agent for treating a hyperlipemia) (which is in the form of the same pharmaceutical preparation such as a mixture, or a mistura), and/or
in combination with at least one agent selected from an antihypertensive agent, a vasodilating agent and an anti-hyperlipidemic agent.

When the postprandial blood sugar-lowering agent is used in combination with at least one agent selected from an antihypertensive agent, a vasodilating agent and an anti-hyperlipidemic agent, they can be administered to a living subject at the same time or at the different times having an appropriate lapse of time between one administration and an another administration, each in a discrete and separate preparation form (They are in the form of plural pharmaceutical preparations.). In such case, there is no limitation to the order (turn) of the preparations for administration.

The present invention is directed to these medicaments and the drug medicine as one embodiment thereof, and they are combined together and generically referred to as "the medicament in the present invention".

Next, the present invention lies in a use of the medicament as an another embodiment thereof, which can be exemplified typically or representatively by the followings:
(4) A method for preventing, improving and/or treating a diabetic complication, which comprises administering a postprandial blood sugar-lowering agent as an effective ingredient (active ingredient) to a living subject; and
(5) A method for preventing, improving and/or treating a neuropathy, which comprises administering a postprandial blood sugar-lowering agent as an effective ingredient to a living subject.

Further, the present invention lies in a use of the postprandial blood sugar-lowering agent for the production of the medicament in the present invention described above as another embodiment thereof, which can be exemplified typically or representatively by the followings:
(6) A use of a postprandial blood sugar-lowering agent for production of the medicament for prevention, improvement and/or treatment of a diabetic complication;
(7) A use of a postprandial blood sugar-lowering agent for production of the medicament for prevention, improvement and/or treatment of a neuropathy; and
(8) Uses of a postprandial blood sugar-lowering agent and at least one agent selected from an antihypertensive agent, a vasodilating agent and an anti-hyperlipidemic agent, for production of the medicament for prevention, improvement and/or treatment of a diabetic complication.

Incidentally, in case of the method for preventing, improving and/or treating a diabetic complication described above, the effectiveness thereof can be enhanced much more by administering further at least one agent selected from an antihypertensive agent, a vasodilating agent and an anti-hyperlipidemic agent, in combination with the postprandial blood sugar-lowering agent to said living subject (Combination administration).

In such case, there is no particular limitations to the method in such combination administration, for example, the plural preparations having each ingredient described above can be administered to the same living subject at the same time or at the different times having an appropriate lapse of time between one administration and an another administration, each in a discrete and separate preparation form. There is no particular limitations to the order (turn) of the preparations for administration.

Further, in the use of a postprandial blood sugar-lowering agent for production of the medicament for prevention, improvement and/or treatment of a diabetic complication described above, as stated above, at least one agent selected from an antihypertensive agent, a vasodilating agent and an anti-hyperlipidemic agent can be mixed with the said postprandial blood sugar-lowering agent for use, or the postprandial blood sugar-lowering agent can be used in the combination form with at least one agent selected from an antihypertensive agent, a vasodilating agent and an anti-hyperlipidemic agent, whereby the effectiveness thereof can be enhanced much more. There are also no particular limitations to the method for such combination, in the same manner as above.

### Brief Description of the Drawings

[Fig. 1]

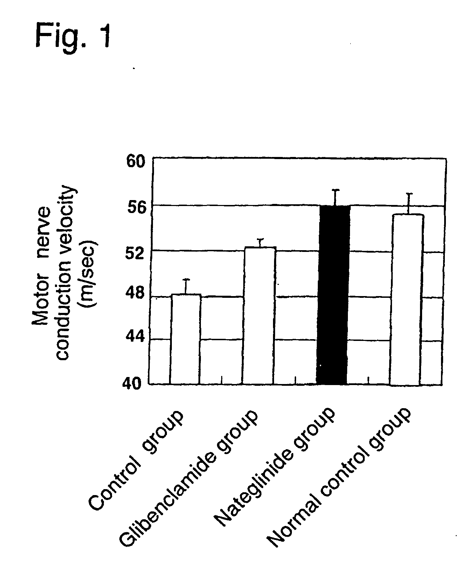

   Fig. 1 illustrates the results of measurement of the motor nerve conduction velocity in the animal test in Example 1.
[Fig. 2]

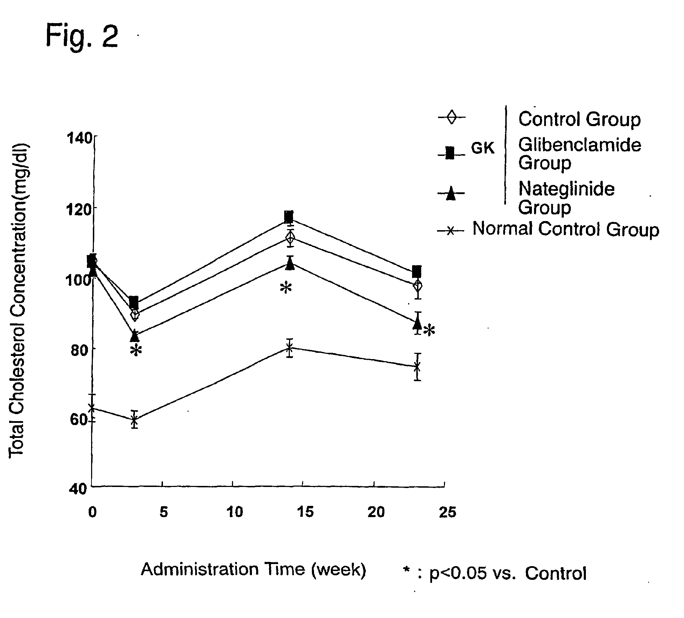

   Fig. 2 illustrates the results of measurement of the change of plasma cholesterol concentration in the animal test in Example 3.

- ◇:: GK rat, control group;
- ■:: GK rat, glibenclamide group;
- ▲:: GK rat, nateglinide group, and
- ×:: normal control group.

### Mode for Carrying out Invention

The followings describe the mode for carrying out the present invention.

The present invention, as stated above, relates to a specified medicament and a specified drug medicine (the medicament(s) in the present invention), uses of these medicaments (method for use thereof, method for administration thereof, etc.), a use of the postprandial blood sugar-lowering agent for production of these medicaments, or the like, and since it is possible to say that the medicament(s) is a common and important factor (constituent element) in the present invention, based on the medicament (the medicament in the present invention) the present invention is explained in the followings.

As for the medicament in the present invention, a purposive medicament in itself may be a drug medicine in itself.

The subject to which the medicament in the present invention is administered is not limited particularly, and typically exemplified by a living subject so far as it seeks for prevention, improvement, treatment and so on of diabetic complication or neuropathy. The medicament (preparation) is applicable in an effective amount thereof to a living body in need of the medicament therefor, in particular a mammal, and usually a human (patient). A method for application of the medicament (method for administration thereof) is corresponding to a method for preventing, improving and/or treating a diabetic complication or neuropathy in the present invention. On the other hand, a use of the postprandial blood sugar-lowering agent which will be explained in detail in the next place as an essentially effective ingredient in the medicament described above, that is, the medicament in the state comprising as an essentially effective ingredient the postprandial blood sugar-lowering agent substantially equal to the above is corresponding to the use of the postprandial blood sugar-lowering agent for production of the medicament for prevention, improvement and/or treatment of a diabetic complication or neuropathy, in the present invention.

The effective component (ingredient) used in the medicament for the present invention is not specifically limited so far as it is used as a postprandial blood sugar-lowering agent, may be used for that purpose (for the purpose of lowering a postprandial blood sugar), and exhibits such an effect. In the present invention, therefore, it is possible to use, as the effective component of the medicament (pharmaceutical preparation) in the present invention, compound (s) selected from those which are used or available for improving the postprandial blood sugar level. The compound(s) that will be developed in the future may be used. There is no particular difficulty in determining whether a compound as a candidate for the ingredient for the objective medicament is applicable as a postprandial blood sugar-lowering agent or not; for example, it can also be determined in such a way that the compound is administered before giving a meal, the blood sugar level is determined 2 hours after the meal, and an inhibitory effect against an elevation of the blood sugar level is evaluated as compared with the case in which no compound (candidate for the ingredient for the objective medicament) is administered. Additionally, it is convenient to utilize, as a component of the medicament in the present invention, one that has already been developed as a different drug medicine, for example, nateglinide or the like, which is described in detail hereinafter.

Specific examples of the postprandial blood sugar-lowering agent include compounds disclosed as meglitinides in *Hormone and Metabolic Research*, Vol. 27, p. 263-266 (1995) and exhibiting such an activity (improving the blood sugar level after meals); more definitely, D-phenylalanine derivatives such as (-)-N-(trans-4-isopropylcyclohexane-carbonyl)-D-phenylalanine represented by the following general formula (1)(called "nateglinide") (see Japanese Patent Kokoku Publication JP-B-4-15221, Japanese Patent No. 2,508,949, and Japanese Patent Kokai Publication JP-A-10-194969, etc.), benzylsuccinic acid derivatives such as mitiglinide (KAD-1229), and benzoic acid derivatives such as repaglinide are enumerated.

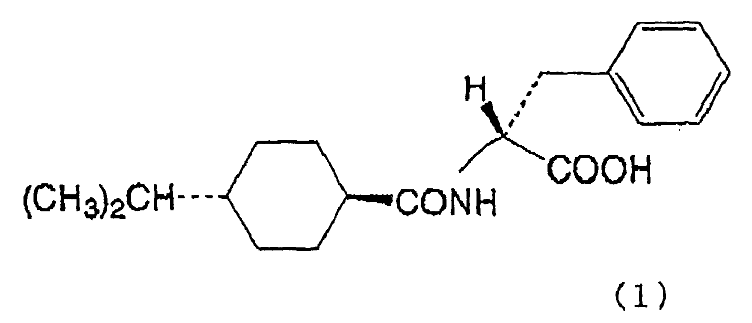

Of the aforementioned postprandial blood sugar-lowering agents, the compounds included in the meglitinides exhibiting an excellent effect in oral administration, are preferred. More preferred are D-phenylalanine derivatives such as nateglinide, benzylsuccinic acid derivatives such as mitiglinide, and benzoic acid derivatives such as repaglinide, are exemplified, with nateglinide being particularly preferred.

The administration form for the medicament in the present invention to a human or the like is not particularly limited. Accordingly, a variety of administration forms such as oral administration and parenteral administration (intravenous administration, etc.) can be employed, and the effective ingredient (component) of the medicament in the present invention is available as described above; when the effective component in the present invention is selected from those which have already been known as postprandial blood sugar-lowering agents, or even when the pharmaceutical component used in the present invention is employed separately, a compound having a postprandial blood sugar-lowering action may be selected and used regardless of any administration form such as oral administration, parenteral administration, and so on. However, it is conventional to employ a medicament available for oral administration.

When the medicament in the present invention is used for a diabetic complication, for example, when the medicament is administered for prevention, improvement and/or treatment of a diabetic complication, there is no particular limitation in the type of complication, and it can widely be applied to nephropathy, retinopathy, neuropathy and angiopathy, etc. On the other hand, when the above medicament is used for treatment or the like in neuropathy, it can also be used widely in a variety of neuropathies as far as it is neuropathy.

In the present invention, the medicament (pharmaceutical preparation) may be used together with other one or more pharmaceutical components (pharmaceutically active substances), for example, as admixture or in combination; in such a case, they are included in the medicament in the present invention as far as they contain an aimed effective component and exhibit the aforementioned aimed pharmacological activity.

Further, such other pharmaceutical component(s) may be in the salt form(s) or the derivative(s) thereof, and also in the salt form(s) or the compounded form(s) with the above essential and effective component(s) as objective in the present invention, and they are included in the medicament(s) in the present invention as far as they exhibit the aforementioned aimed pharmacological activity for the present invention described above.

Such pharmaceutical components, for example, are exemplified by an insulin exhibiting a blood sugar-lowering action, an insulin derivative such as lispro and glargine, a sulfonylurea drug (sulfonylureas) such as tolbutamide, gliclazide, glibenclamide and glimepiride, a α-glucosidase inhibitor such as acarbose, voglibose and miglitol, a biguanide preparation such as metformin and phenformin, a thiazolidinediones such as pioglitazone, rosiglitazone and troglitazone, an insulin resistance-improving agent containing PPARγ agonist and antagonist of non-thiazolidine skeleton such as GI-262570, JTT-501, YM-440, NN-622 and KRP-297, an adrenaline β3 receptor agonist such as AJ-9677, an insulin like agonist such as CLX-0901, a GLP-1 agonist such as GLP-1, Exendin-4 and NN-2211, a DPPIV inhibitor such as DPP-728A, a SGLT inhibitor such as T-1095, and the like. And, also an aldose reductase inhibitor such as epalrestat, fidarestat, zenarestat and minalrestat, a neuropathy-treating agent such as mecobalamin, mexiletin and Y-128, and an antioxidant agent such as α lipoic acid and probucol, which are said to have an effect to the complication, may be cited therefor.

Particularly, a postprandial blood sugar-lowering agent capable of suppressing a vascular dysfunction may be used in a combination with, or in a mixture with;
an antihypertensive agent capable of lowering a blood pressure, for example, angiotensin converting enzyme inhibitor such as captopril, delapril, alacepril, enalapril, lisinopril, cilazapril, benazepril, imidapril, temocapril, quinapril, trandolapril and perindopril, an angiotensin II receptor (reipient) antagonist such as losartan, candesartan, irbesartan and valsartan, a β blocker such as bopindolol, pindolol, carteolol, propranolol, nadolol, nipradilol, acebutolol, celiprolol, metoprolol, atenolol, bisoprolol, betaxolol, labetalol, carvedilol, bevantolol, amosulalol and arotinolol, an α 1 blocker such as prazosin, bunazosin, terazosin, doxazosin and urapidil, a calcium channel blocker (calcium antagonist) such as nifedipine, nicardipine, nilvadipine, nitrendipine, nisoldipine, manidipine, benidipine, barnidipine, amlodipine, efonidipine, felodipine, cilnidipine, aranidipine and diltiazem, a diuretic drug such as cyclopenthiazide, trichlormethiazide, hydrochlorothiazide, hydroflumethiazide, benzylhydrochlorothiazide, methyclothiazide, indapamide, chlortalidone, tripamide, meticrane, metolazone, mefruside, azosemide, etacrynic acid, piretanide, bumetanide, furosemide, spironolactone and triamterene, and the like, in the form of combination preparation or mixed preparation. Such combination or mixture is effective for a diabetic complication, in particular, a microangiopathy such as a nephropathy, a neuropathy and a retinopathy.

In the same manner, a postprandial blood sugar-lowering agent capable of suppressing an angiopathy (disorder of blood vessel) may be used in a combination with, or in a mixture with;
a vasodilating agent, for example, a prostaglandin derivative (preparation) such as beraprost and alprostadil, a serotonin receptor antagonist such as ketanserin, sarpogrelate and AT-1015, a phosphodiesterase inhibitor such as cilostazol, a COX inhibitor such as various anti-platelet agents (for example, aspirin), a thromboxane synthetic enzyme (synthetase) inhibitor such as ozagrel, an ADP receptor (recipient) inhibitor such as ticlopidine and clopidogrel, and others; pentoxifylline, eicosapentaenoic acid, tocopherol nicotinate, etc., in the form of combination preparation or mixed preparation. Such combination or mixture also is effective for a diabetic complication, in particular, a microangiopathy such as a nephropathy, a neuropathy and a retinopathy.

In addition, a postprandial blood sugar-lowering agent capable of lowering a lipid in the blood may be used in a combination with, or in a mixture with;
an anti-hyperlipidemic agent, for example, a HMG-CoA reductase inhibitor such as pravastatin, simvastatin, fluvastatin, cerivastatin, atorvastatin and itavastatin, a fibrate derivatives such as simfibrate, clofibrate, clinofibrate, bezafibrate and fenofibrate, an anion exchange resin such as colestimide and colestyramine (cholestyramine), a nicotinic acid preparation such as nicomol and niceritrol, and the like, in the form of combination preparation or mixed preparation. Such combination or mixture is effective for a diabetic complication, in particular, a cerebral infarct, a heart infarction (myocardial infarct), an arteriosclerosis obliterans, etc. based on a macroangiopathy caused by sclerosis of the arteries.

Besides, a variety of pharmacologically acceptable pharmaceutical substances maybe contained (as adjuvants, etc.), which also may be referred to as "pharmaceutically acceptable carriers". The pharmaceutical substances may be properly selected according to the form of the pharmaceutical preparations, including, for example, excipient, diluent, additive, disintegrator (disintegrating agents), binder, coating agent, lubricant, sliding agent, lubricating agent (lubricant pharmaceuticals), flavor, sweetener, solubilizing agent, and the like. Additionally, specific examples of the pharmaceutical substances include magnesium carbonate, titanium dioxide, lactose, mannitol and other sugars (saccharide), talc, cow's milk proteins, gelatin, starch, cellulose and its derivatives, animal and vegetable oils, polyethylene glycol, and solvents such as sterile water and mono- or polyhydric alcohols, e.g., glycerol.

The medicament in the present invention, as mentioned above, may be prepared into a variety of pharmaceutical formulations which are known or will be developed in the future, for example, various forms for administration such as oral administration, intraperitoneal administration, percutaneous administration, inhalation administration, and so on. In order to prepare the medicament in the present invention into a form of a variety of these pharmaceutical formulations (preparations), it is possible to apply properly a known method or a method developed in the future.

As a form of a variety of these pharmaceutical formulations, for example, a form of suitable solid or liquid formulations such as granules, powders, coated tablets, tablets, (micro)capsules, suppositories, syrups, juices, suspensions, emulsions, dropping preparations, solutions for injection, formulations for retarding release of the active substance, and the like are exemplified.

It is natural that the medicament in the present invention being in a form of the pharmaceutical formulation as exemplified above should comprise an effective amount of the aforementioned component (ingredient; postprandial blood sugar-lowering agent) to exhibit the pharmacological (medicable) effect (or drug efficacy).

The dose of the pharmaceutical medicament (the ingredient) in the present invention is suitably selected depending on the type of postprandial blood sugar-lowering agent, the variety of complication, the degree of the symptom in complication or neuropathy, the form of pharmaceutical formulation, the presence or absence of side effect or the degree thereof, and the like. In a case of the pharmaceutical formulation containing nateglinide as an effective component, it may be administered to a patient, in terms of the net weight of nateglinide, preferably in a daily oral dose of from about (approximately) 10 mg to 10 g, more preferably from about 30 mg to 1 g, further more preferably from about 90 to 270 mg. In a severe case, it may be increased. With respect to frequency and timing of doses, though once several days or once a day may also be acceptable, usually, it may be administered several times a day, for example, in from 2 to 4 divided doses, preferably before meals. In the case of intravenous administration, from about one tenth to one twentieth may be acceptable in comparison with the dose of the aforementioned oral administration.

From the above explanations, in case that the medicament in the present invention is used in the method for preventing, improving and/or treating a diabetic complication or neuropathy in the present invention, the method for using the said medicament is easily understood.

More simply, for the postprandial blood sugar-lowering agent used in the present invention, the compound (drug medicine) developed already or known under development or the like, and so on as the postprandial blood sugar-lowering agent or an agent having the same effect thereto, can be employed. Depending on the type of the postprandial blood sugar-lowering agent, an amount used (consumed) thereof and an amount thereof for administration suitable in an effective amount of each medicament can be employed. All the contents described or reported publicly on the type of such medicaments, the method for use thereof, the amounts thereof for administration, etc. are incorporated in the description of this patent application as a part thereof by reference.

As stated above, in addition to the essential ingredient (postprandial blood sugar-lowering agent) employed for the medicament in the present invention, other medicament ingredient(s) can further employed. Even in such case, based on the above explanations or by utilizing a known technique for pharmaceutical preparation (formulation), or according to the form of a variety of formulations (dosage form), a required formulation can be also prepared for such other medicament. As for the amount employed thereof, naturally the medicament (the above other medicament) may be employed in a pharmacologically effective amount to exhibit the drug efficacy in such other medicament. As far as the medicament may be employed in such way, there is also no particular limitations to the amount employed of said other medicament ingredient for administration when such other medicament is used at the same time with the postprandial blood sugar-lowering agent described above (in the mixture form, in the combination form, etc.).
For example, it is suitably selected depending on the type of postprandial blood sugar-lowering agent as the essential ingredient, the variety of complication or the like, the degree of the symptom in complication or neuropathy, the form of pharmaceutical formulation, the presence or absence of side effect or the degree thereof, and the like.

In particular, for example, in case that at least one agent selected from an antihypertensive agent, a vasodilating agent and an anti-hyperlipidemic agent is used in a mixture with, or in a combination with the postprandial blood sugar-lowering agent, as for the amount employed of these medicament(s) (the antihypertensive agent, the vasodilating agent and/or the like) or the amount employed thereof for administration, an amount used (consumed) thereof and an amount thereof for administration suitable in an effective amount of each medicament (drug medicine) developed already or known under development or the like, and so on as the said antihypertensive agent, vasodilating agent or anti-hyperlipidemic agent, and/or an agent having the same effect thereto can be employed.
Accordingly, all the contents described or reported publicly on the type of such known antihypertensive agent, vasodilating agent or anti-hyperlipidemic agent, the type of the agents having the same effect thereto, the methods for use thereof, the amounts thereof for ,and the like are incorporated in the description of this patent application as a part thereof by reference.

### Preferred Mode for Carrying out Invention

Hereinafter, the present invention is explained in detail based on the following Examples. In these Examples, % values are % by weight unless otherwise noted specifically.

### (Example 1)

A motor nerve conduction velocity was measured in an animal test, and an influence on the complication (improving effect) was examined.

### (Test Method)

An animal test was conducted on a set of the following four groups, and nateglinide and glibenclamide were each suspended into 0.5 % methyl cellulose (MC) to give a solution for administration.

| Test group | Animal | Drug administered | Number of animal |
|---|---|---|---|
| Control group | GK rat | 0.5% MC | 8 |
| Glibenclamide group | GK rat | Glibenclamide 1 mg/kg | 8 |
| Nateglinide group | GK rat | Nateglinide 50 mg/kg | 9 |
| Normal control group | Wistar rat | 0.5% MC | 9 |
| (Test Animal) | | | |

GK rats (♂: male) as a spontaneous diabetic model and normal Wistar rats (♂: male) were introduced at the age of 6 weeks.

From the age of 7 weeks, they were kept under a light control (dark term: 7:00 a.m. to 7:00 p.m.; light term: 7:00 p.m. to next morning 7:00) in a feeding limit condition, in which a feed was given twice a day, each only for 1 hour at an interval of 6 hours (9:00 a.m. to 10:00 a.m., and 3:00 p.m. to 4:00 p.m.).

Everyday from the age of 14 weeks, 0.5% MC, glibenclamide (1 mg/kg) or nateglinide (50 mg/kg) was forcibly administered orally just before each feeding twice a day (at 9:00 a.m. and 3:00 p.m.) at an interval of 6 hours.

### (Measurement of Motor Nerve Conduction Velocity)

Twenty-three weeks after the start of the drug administration (the age of 37 weeks), in order to examine an influence on diabetic complications, the motor nerve conduction velocity was measured according to the method described in Cameron et al., Diabetologia, Vol. 39, p.1047-1054 (1996), using a tail nerve. The results are shown in Fig. 1.

### (Results of Evaluation)

As is clear from the results of Fig. 1, in a GK rat (control group) which is a spontaneous diabetic model, a marked decrease of the motor nerve conduction velocity, which is one of indicators of diabetic complication, was observed (control group: 48.2 ± 1.3 m/sec, normal control group: 55.2 ± 1.8 m/sec) in comparison with that of the normal control group.

A slight improvement was observed in a group to which a sulfonylurea drug, glibenclamide used generally as an oral anti-diabetic drug (oral hypoglycemic agent), was administered (52.3 ± 0.9 m/sec). Moreover, the GK rats to which nateglinide was administered retained a motor nerve conduction velocity equal to that of the normal rats (55.9 ± 1.3 m/sec) ; thus, marked effects for prevention, improvement and treatment with nateglinide against onset and progress of neuropathy were demonstrated.

### (Example 2)

### (Measurement of Na⁺/K⁺-ATPase Activity in Nerve)

In the same manners as those in the Example 1, the group formation for the test animals, the feeding of the animals and the administration of medicinal compounds (drug) to be tested to the animals were performed. At twenty-three weeks after the start of the drug administration (the age of 37 weeks), the rats were killed and the sciatic nerves were excised. A Na⁺/K⁺-ATPase activity in the sciatic nerve was measured based on the method described in Green et al., Journal of Clinical Investigation, vol.72, 1058-1063 (1983).

### (Results of Evaluation)

In the GK rats (control group), a marked decrease of the Na⁺/K⁺-ATPase activity in the nerve, which is one of indicators for neurological dysfunction, was observed (control group: 87.6 ± 9.8 µmol ADP/g/hr, normal control group: 110.5 ± 7.7 µmol ADP/g/hr) in comparison with that of the normal control group.

In the GK rats to which nateglinide was administered, the decrease of the Na⁺/K⁺-ATPase activity was suppressed (99.5 ± 7.6 µmol ADP/g/hr); thus, the effects for prevention, improvement and treatment with the nateglinide against onset and progress of neuropathy were suggested.

### (Example 3)

### (Measurement of Total Cholesterol Concentration in Blood Plasma)

In the same manners as those in the Example 1, the groups formation for the test animals, the feeding of the animals and the administration of medicinal compounds (drug) to be tested to the animals were performed. At one week before the start of the drug administration (the age of 13 weeks) for the animals, and at four weeks (the age of 18 weeks), fourteen weeks (the age of 28 weeks) and twenty-three weeks (the age of 37 weeks) each after the start of the drug administration, the blood samples were taken from the jugglar vein after fasting (abstinence from food) for 17 hours. The total cholesterol concentration in the blood plasma was measured by means of the cholesterol oxidase test method using the Fuji Drichem analyzer. The results are shown in Fig. 2.

### (Results of Evaluation)

As is clear from the results of Fig. 2, in a GK rat (control group), a marked increase of the total cholesterol concentration in the blood plasma which is a risk factor to an arteriosclerosis (sclerosis of the arteries) or a macroangiopathy was observed, at any times for the measurement.

A change in the cholesterol value was not observed in the group to which a sulfonylurea drug, glibenclamide used generally as an oral anti-diabetic drug, was administered. On the contrary, in the GK rats to which the nateglinide was administered a marked decrease of the total cholesterol concentration in the blood plasma was observed, and thus the results suggested the effects for prevention, improvement and treatment with the nateglinide against onset and progress of the diabetic complication, particularly an arteriosclerosis and a macroangiopathy.

### (Example 4)

A triglyceride concentration in blood plasma was measured.

### (Test Method)

To the GK rats under fasting (abstinence from food), a fat emulsion (Intralipos, 2 g/kg) is forcibly administered, and thereby an increase of triglyceride in blood plasma exhibiting a peak at two hours after the administration is observed. Using the postprandial hyperlipemia model rats (10 rats) thus obtained, in a crossover method, an influence of the drug administered on a triglyceride concentration in blood plasma (a postprandial hyperlipemia- suppressing effect) was examined. That is, nateglinide (50 mg/kg) or voglibose (0.2 mg/kg), an alpha-glucosidase inhibitor as a control was administered orally, just before loading with the fat emulsion, and the blood samples were taken from a tail vein with time by four hours after the administration.

### (Measurement of Triglyceride Concentration in Blood Plasma)

A triglyceride concentration in the blood plasma was measured by means of the Fuji Drichem analyzer.

### (Results of Evaluation)

An increase of area under the curve ( 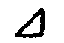 AUC) of the triglyceride during four hours after the loading, in the voglibose administration group was 164 ± 17 mg·h/dl. On the contrary, in the group to which the nateglinide was administered , a marked decrease of the 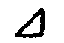 AUC was observed (81 ± 22 mg^{·}h/dl).

### (Results of Evaluation with Zucker Fatty Rat)

The same examinations were also conducted with the use of a Zucker Fatty rat which is another hyperlipidemic animal. The Zucker Fatty rat exhibits a marked postprandial hyperlipemia under load of the fat emulsion much more than the GK rat. The area under the curve (ΔAUC) of the triglyceride until four hours after the load thereof, in the voglibose- administration group was 501 ± 112 mg^{·}h/dl. On the contrary, in the group to which the nateglinide was administered, a marked decrease of the 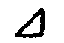 AUC was observed (15 ± 69 mg^{·}h/dl).

As a result of fractional analysis of lipoprotein by means of an agarose gel electrophoresis, a marked increase of fractions in the origin area and pre β subfractions, which are thought to contain chylomicron, VLDL (very low density lipoprotein) and so on which are said to correlate with an angiopathy and an arteriosclerosis was observed. In the group to which the nateglinide was administered, such increases were suppressed, and thus the result also suggested the effect for prevention, improvement and treatment with the nateglinide against onset and progress of the diabetic complication, particularly an arteriosclerosis and a macroangiopathy.

### (Example 5)

### (Measurement of vWF Concentration in Serum)

In the same manners as those in the Example 1, the feeding of the animals for the test animals and the administration of medicinal compounds (drug) to be tested to the animals were performed. At twenty-three (23) weeks after the start of the drug administration (the age of 37 weeks), the blood samples were taken from the postcaval vein. The vWF concentration in the serum was measured by means of the ELISA method using anti-human vWF antibody (made by DAKO Co.).

### (Results of Evaluation)

In the GK rats (control group), a marked increase of a vWF concentration in the serum which is one of the indicators for vascular endothelial disorder was observed (control group: 184.8 ± 24.3 %, to normal control group: 100 (± 22.7 %)) in comparison with that of the normal control group.

On the contrary, in the GK rats to which nateglinide was administered, the increase of the vWF concentration in the serum was suppressed (124.5 ± 21.5%); thus the effects for prevention, improvement and treatment with the nateglinide against onset and progress of the angiopathy were suggested.

As is clear from the foregoing results, it is confirmed that a decrease of the motor nerve conduction velocity in the diabetic rats is markedly improved by administration of nateglinide; thus, it is suggested that nateglinide is superior (excellent) as an effective ingredient in a pharmaceutical preparation for a diabetic complication, as well as that it also has a potentiality to be used as an effective ingredient in a pharmaceutical preparation for prevention, improvement and/or treatment of a neuropathy independent of the diabetic complication, and so it is expected to be a pharmaceutical preparation directed towards each of them.

In addition, it was confirmed that a decrease of Na⁺/K⁺-ATPase activity in the nerve, an increase of the vWF concentration in the serum and an increase of the total cholesterol concentration in the blood plasma, in the diabetic rats are all markedly improved by administration of the nateglinide. In view of the above confirmation, also the nateglinide may be expected strongly for a medicament (pharmaceutical preparation) used for preventing, improving and/or treating a diabetic complication and/or a neuropathy.

### Effects of Invention

According to the present invention, a medicament useful in prevention, improvement, treatment, etc. of diabetic complication, a medicament useful in prevention, improvement, treatment, etc. of neuropathy, a method for use thereof (a method for administration thereof in treatment, etc. thereof and the like), a use of the postprandial blood sugar-lowering agent for production of said medicaments therefor, and the like can be provided.

In particular, D-phenylalanine derivatives such as nateglinide, benzylsuccinic acid derivatives such as mitiglinide, and benzoic acid derivatives such as repaglinide are much expected as an oral administration preparation (oral medicine).

In addition, one or more of an antihypertensive agent, a vasodilating agent, an anti-hyperlipidemic agent, etc. may be used;
in a mixture with (in the form of the same preparation) , or in a combination with (in the form of plural pharmaceutical preparations) the postprandial blood sugar-lowering agent as the above essential ingredient, whereby the effect for prevention, improvement, treatment, etc. of the diabetic complication described above can be enhanced much more.

## Claims

1. A medicament for prevention, improvement and/or treatment of a diabetic complication, comprising a postprandial blood sugar-lowering agent, which may comprise pharmaceutically acceptable carrier(s).

2. The medicament as defined in claim 1, wherein said diabetic complication is at least one of nephropathy, retinopathy, neuropathy and angiopathy.

3. The medicament as defined in claim 1 or 2, wherein said postprandial blood sugar-lowering agent is a compound included in meglitinides.

4. The medicament as defined in claim 3, wherein said compound included in meglitinides is nateglinide.

5. The medicament as defined in any one of claims 1 to 3, which is meant for an oral administration.

6. A medicament for prevention, improvement and/or treatment of a neuropathy, comprising a postprandial blood sugar-lowering agent, which may comprise pharmaceutically acceptable carrier(s).

7. The medicament as defined in claim 6, wherein said postprandial blood sugar-lowering agent is a compound included in meglitinides.

8. The medicament as defined in claim 7, wherein said compound included in meglitinides is nateglinide.

9. The medicament as defined in claim 1, further comprising at least one agent selected from an antihypertensive agent, a vasodilating agent and an anti-hyperlipidemic agent.

10. The medicament as defined in claim 1, which is used in combination with at least one agent selected from an antihypertensive agent, a vasodilating agent and an anti-hyperlipidemic agent.

11. A drug medicine suitable or usable for the medicament for prevention, improvement and/or treatment of a diabetic complication,
comprising a postprandial blood sugar-lowering agent and at least one agent selected from an antihypertensive agent, a vasodilating agent and an anti-hyperlipidemic agent; or
being a combination of a postprandial blood sugar-lowering agent, with at least one agent selected from an antihypertensive agent, a vasodilating agent and an anti-hyperlipidemic agent, and
which may comprise or have pharmaceutically acceptable carrier(s).

12. A method for preventing, improving and/or treating a diabetic complication, which comprises administering a postprandial blood sugar-lowering agent to a living subject.

13. A method for preventing, improving and/or treating a neuropathy, which comprises administering a postprandial blood sugar-lowering agent to a living subject.

14. The method as defined in claim 12, which further comprise administering at least one agent selected from an antihypertensive agent, a vasodilating agent and an anti-hyperlipidemic agent, to said living subject.

15. A use of a postprandial blood sugar-lowering agent for production of the medicament for prevention, improvement and/or treatment of a diabetic complication.

16. A use of a postprandial blood sugar-lowering agent for production of the medicament for prevention, improvement and/or treatment of a neuropathy.

17. The use as defined in claim 15, wherein said postprandial blood sugar-lowering agent is used in the state of the mixture with at least one agent selected from an antihypertensive agent, a vasodilating agent and an anti-hyperlipidemic agent.

18. The use as defined in claim 15, wherein said postprandial blood sugar-lowering agent is meant for use in combination with at least one agent selected from an antihypertensive agent, a vasodilating agent and an anti-hyperlipidemic agent.

19. Uses of a postprandial blood sugar-lowering agent and at least one agent selected from an antihypertensive agent, a vasodilating agent and an anti-hyperlipidemic agent, for production of the medicament for prevention, improvement and/or treatment of a diabetic complication.
